# EUROPEAN PATENT SPECIFICATION

(11) **EP 1 991 130 B1**
(45) Date of publication and mention of the grant of the patent: **22.08.2012**
(21) Application number: 07705319.7
(22) Date of filing: 02.03.2007
(51) Int. Cl.: A61B 10/00

(54) **SAMPLE COLLECTION AND TESTING DEVICE WITH PIVOT ARM**
PROBENNAHME- UND PRÜFVORRICHTUNG MIT SCHWENKARM
DISPOSITIF DE PRÉLÈVEMENT ET D'ANALYSE D'ECHANTILLONS COMPORTANT UN BRAS PIVOTANT

(30) Priority: 03.03.2006 GB 0604329
(43) Date of publication of application: 19.11.2008
(73) Proprietor: Systagenix Wound Management IP Co. BV., 1077 XX Amsterdam (NL)
(72) Inventor: HANNANT, Matthew, London, SW8 3RL (GB); IRWIN, Stephen, James, London W5 2RB (GB); BAYLIFF, Simon, William, North Yorkshire BD23 6AH (GB)
(74) Representative: James, Anthony Christopher W.P.
(86) International application number: PCT/GB2007/000739
(87) International publication number: WO 2007/099344

(56) References cited:
- EP-A- 0 570 867
- US-A- 5 980 828
- US-A1- 2004 171 173

## Description

The present invention relates to sample collection and testing devices, in particular to devices for collecting and testing biological samples such as wound fluid.

It is known to collect samples, such as biological samples, by using a sample collection device comprising a swab mounted on a shaft. Analysis of the collected sample may then be undertaken in a separate testing device, which includes a sample inlet port for receiving a sample from the swab and an analytical device capable of indicating whether the sample has tested positive for a particular predetermined analyte.

US patents 5,266,266 and 6,248,294 describe self-contained diagnostic swab units comprising a swab mounted on a hollow shaft, and a tubular housing for receiving and storing the swab. The tubular housing further comprises a chamber having diagnostic test reagents contained therein for analysing a sample from the swab. The distal end of the hollow shaft communicates with a reservoir of liquid, which can be expelled through the hollow shaft to flush a sample from the swab. Following collection of a sample, the swab is reinserted into the tubular housing, and the sample is flushed from the swab into the chamber for analysis.

The above devices all comprise at least two elongate parts, which are separated in use. Furthermore, the transfer of the sample from the swab to the analysis device is inefficient.

US-A-2004/0171173 describes a swab connected to a housing that incorporates a test strip. The swab is mounted at the distal end of a swab arm. Front and back sides of the housing are hinged about the proximal end of the swab arm, whereby they can be pivoted to enclose the swab before or after use. An analytical test strip may be provided in one of the sides of the housing, whereby a liquid sample in the swab is squeezed onto the test strip when the housing is closed about the swab. The housing may also contain a reservoir of buffer liquid to wash the sample onto the test strip. A window may be provided in the housing to permit viewing of the test strip when the housing is closed. These assemblies require a double hinge between the housing parts and the swab arm.

Moreover, it is difficult to manipulate the assembly between the closed and open positions without contaminating the swab and/or the inside of the housing.

WO01/29558 describes a test strip incorporating a pre-treatment chamber. The pre-treatment chamber is formed from a hinged extension to the test strip holder. The device does not incorporate a swab. In use, a sample is introduced into a sample receiving port in the pre-treatment chamber.

EP-A-0570867 describes a bioassay device comprising a well at the distal end of an arm. The proximal end of the arm is hinged to a collection section containing an absorbent material. A microporous membrane comprising a receptor for a target ligand extends across the bottom of the well. In use, a liquid sample is placed in the well, and the hinged arm is then folded back to bring the bottom of the microporous membrane into contact with an absorbent material to draw the sample through the membrane. The target ligand is bound to the microporous membrane, where it can be analysed for example by applying a labelled second receptor for that ligand. The device does not incorporate a swab.

US2002/0004019 describes a saliva testing device incorporating a hinged expresser cup adapted to receive and compress a separate foam collection swab.

US-A-5747351 describes a chromatographic assay test device incorporating a test strip and a sample collection member that is constructed from five interconnected panels which are die-cut from cardboard. The device does not incorporate a swab.

US patent 5,980,828 describes a combined sample-assay device that is a flat plate-like body with a collection arm. The collection arm folds to enter a recess that flatly supports and seats the arm. US '828 discloses the features of the preamble of claim 1.

According to the present invention, there is provided a sample collection and testing device as defined in claim 1.

The inventive device allows a user to collect a sample and then to conduct testing on that sample within a compact, unitary hand-held device. This enables single-handed, simple and efficient operation. It also avoids the possibility of component parts of a sample collection and testing kit becoming separated from one another. Moreover, since the sample collector can be pivoted into the sample receiving port within the housing, the possibility of contamination of the sample collector between the taking of a sample and the testing of that sample is reduced. Furthermore, the design of the device permits highly efficient transfer of a sample from the collector device to the analytical device, as will be seen in more detail below.

Unlike the devices of US-A-2004/0171173, the pivot arm is located substantially externally of the housing in both the sample collection and the storage/analysis configurations, thereby reducing the risk of contamination of the analytical device in use. Since only the pivot arm is moved between these configurations (the configuration of the housing remaining fixed), the operation and construction of the device are simplified.

### The Housing

Suitably, the housing is an elongate housing having a principal axis. The housing may be made up of one, two, or more parts, for example assembled by snap-fitting. The housing is adapted to receive the analytical device. For example, the analytical device may be received in a chamber inside the housing. In other embodiments, the analytical device may be attached to a side of the housing. The housing is suitably shaped to provide a convenient handpiece for sample collection, for example an elongate housing.

The housing may be at least partially transparent, or may have windows provided therein, for observation of at least a zone of the analysis device that undergoes a color or fluorescence change inside the housing.

### The pivot arm and sampling device

The pivot arm is likewise suitably formed from thermoplastics, for example by injection molding. Suitably, the proximal end of the pivot arm is pivotably attached to the housing by a hinge. That is to say, by a pivot that allows rotation of the pivot arm in a single plane.

Suitably, the sample collector at the distal end of the pivot arm is a swab, that is to say a small pad of liquid-absorbent material. In certain alternative embodiments, the sample collector may comprise a biopsy punch, pipette, or other mechanical sampling device.

Suitably, the pivot arm is integral with that housing. That is to say, it is molded in one piece with at least one component of the housing. This aids the manufacturing process and ensures that the pivot arm does not become separated from the housing. In these embodiments, a hinge connection between the pivot arm and the housing may be formed by a thin section of the molding material.

In embodiments wherein the housing is an elongate housing, the pivot arm is suitably pivoted proximate to a first end of the housing. In these embodiments, the pivot arm, when in the sample collection configuration, is suitably substantially coaxial with the said principal axis. Suitably, the length of the pivot arm is at least about 50% of the length of the housing, more suitably at least about 75% of said length of the housing.

When the pivot arm is folded in the sample analysis configuration it is folded back along (but not necessarily in contact with) an outer surface of the housing, but is not enclosed within the housing. The sample collector, when the pivot arm is folded in the sample analysis configuration, is located in the sample receiving port of the housing. In certain embodiments, the sample collector is then brought into direct contact with the analytical device. In other embodiments, the sample receiving port is in fluid communication with the analytical device by means of a conduit in the housing, or by means of a capillary transfer device such as a wick. With such an arrangement, the collected sample may be taken up from the sample collector into the analytical device with minimum dead volume and minimum dilution of the sample.

Suitably, the device further comprises complementary detent elements associated with the pivot arm and the housing to retain the pivot arm in the sample collection configuration until a predetermined minimum pivoting force is applied to the pivot arm. The detents may engage by snap-fitting, whereby application of a sufficient torque can overcome the engagement after collection of the sample, in order to fold the pivot arm into the analysis configuration.

Additionally, the device may further comprise complementary detent elements associated with the pivot arm and the housing to retain the pivot arm in the analysis configuration. The detents may engage by snap-fitting, and in certain embodiments application of a sufficient torque can overcome the engagement to allow unfolding of the pivot arm into the sample collection configuration.

### The retaining clip and reservoir

Alternatively or additionally, the device according to the present invention may comprise a retaining clip to retain the pivot arm in the sample analysis configuration. Suitably, the retaining clip is pivotally attached to the housing and is pivotable from a free configuration to a retaining configuration in which at least a portion of the clip engages the distal end of the pivot arm. Suitably, where the device includes a retaining clip, the clip is integral with at least one part of the housing. For example, the hinge between the clip and the housing may be formed by a thin region of the molding plastic. This aids the manufacturing process and ensures that the retaining clip does not become separated from the housing.

In certain embodiments, at least a portion of the retaining clip in said retaining configuration overlies the distal end of the pivot arm and the sample receiving port. Such an arrangement ensures that once the pivot arm has been moved to the sample analysis configuration, it is retained there with at least a portion of the clip overlying the sample receiving port. The possibility of contamination of the sample collector is thus further reduced.

Suitably, the device according to the present invention further comprising a liquid reservoir for releasing a liquid onto the sample collector when the device is in the sample analysis configuration. The liquid may have a number of functions. Primarily, it washes the sample out of the sample collector and into the analytical device. It also functions as a diluent for the sample. It may also contain reagents for interacting with the sample, as described further below.

The liquid reservoir suitably contains from about 0.05ml to about 1ml of liquid, for example from about 0.1 to about 0.5ml of liquid. It is preferably located proximate to the sample receiving port. The close proximity of the reservoir, the sample receiving port, the sample collector and the analytical device when the apparatus according to the present invention is in the analysis configuration is a significant advantage, as it minimises the amount of liquid needed to transfer the sample onto the analytical device.

In certain embodiments, the liquid reservoir is mounted on a retaining clip attached to the housing, as hereinbefore described. In a preferred embodiment, the liquid reservoir is mounted on a portion of the retaining clip that overlies the sample receiving port when the clip is in the retaining configuration. This provides a short fluid flow path from the reservoir to the analytical device.

The liquid reservoir is substantially sealed, but comprises at least one element that allows the reservoir to be opened to release the liquid onto the sample collector when the apparatus is in the analysis configuration. Suitably, the reservoir comprises a zone of weakness in fluid communication with the sample collector when the apparatus is in the analysis configuration. The reservoir may be compressible, in which case application of pressure (e.g. finger pressure) to the reservoir may be sufficient to rupture the zone of weakness and release the liquid. Alternatively or additionally, the device may comprise a projection on the housing or on the pivot arm that ruptures the zone of weakness when the apparatus is folded into the sample analysis configuration. In yet other embodiments, the reservoir may comprise an opening that is covered by a seal that can be opened by hand immediately before the apparatus is folded into the sample analysis configuration, for example a peelable sealing sheet.

### The analytical device

The analytical device in the apparatus of the present invention may be any device that produces a detectable signal in response to one or more predetermined analytes. The signal may observable or measurable by a physical, chemical, or biological means known to those of skill in the art. A detectable signal may be a change in emission or absorbance of electromagnetic waves at a certain wavelength, hybridization or enzymatic reaction. In preferred embodiments, detectable signals are changes in colour when viewed under white light, or fluorescence when viewed under UV light. In certain embodiments, the device may be used in conjunction with an electronic sensor, for example to detect color change or fluorescence and to provide a quantitative output thereof. The electronic sensor can provide a quantitative output in digital form.

In certain embodiments, the analytical device operates on the lateral flow principle. By "lateral flow", it is meant liquid flow in which the dissolved or dispersed components of the sample are carried, preferably at substantially equal rates, and with relatively unimpaired flow, laterally through a carrier. Suitably, the fluid flow path contains one or more porous carrier materials. The porous carrier materials are preferably in fluid communication along substantially the whole fluid flow path so as to assist transfer of fluid along the path by capillary action. Suitably, the porous carrier materials are hydrophilic, but preferably they do not themselves absorb water. The porous carrier materials may function as solid substrates for attachment of reagents or indicator moieties.

The size and shape of the carrier are not critical and may vary. The carrier defines a lateral flow path. Suitably, the porous carrier is in the form of one or more elongate strips or columns. In certain embodiments, the porous carrier is one or more elongate strips of sheet material, or a plurality of sheets making up in combination an elongate strip. One or more reaction zones and detection zones would then normally be spaced apart along the long axis of the strip. However, in some embodiments the porous carrier could, for example be in other sheet forms, such as a disk. In these cases the reaction zones and detection zones would normally be arranged concentrically around the center of the sheet, with a sample application zone in the center of the sheet. In yet other embodiments, the carrier is formed of carrier beads, for example beads made from any of the materials described above. The beads may suitably be sized from about 1 micrometer to about 1mm. The beads may be packed into the flow path inside the housing, or may be captured or supported on a suitable porous substrate such as a glass fiber pad.

It will be appreciated that the devices according to the present invention may be adapted to detect more than one analyte. This can be done by the use of several different reagents in a single reaction zone, or preferably by the provision in a single device of a plurality of flow paths each adapted for detecting a different analyte. In some embodiments, the plurality of fluid flow paths are physically separated within the housing. In other embodiments multiple flow paths (lanes) can be defined in a single strip by depositing lines of wax or similar hydrophobic material between the lanes.

The devices according to the present invention may for example incorporate a bacterial sensing device of the kind described in copending application GB 0501818.9 filed on 28th January 2005.

An absorbent element may suitably be included in the devices of the present invention. The absorbent element is a means for drawing the liquid sample through the device by capillary attraction. Generally, the absorbent element will consist of a hydrophilic absorbent material such as a woven or nonwoven textile material, a filter paper or a glass fiber filter.

The device may further comprise at least one filtration element intermediate the sample receiving port and the analytical device to remove impurities from the sample before the sample undergoes analysis. The filtration device may for example comprise a microporous filtration sheet for removal of cells and other particulate debris from the sample.

In certain embodiments, the devices according to the present invention include a control moiety in a control zone of the device, wherein the control moiety can interact with a component of the sample to improve the accuracy of the device. Suitably, the control zone is adapted to reduce false positive or false negative results. A false negative result could arise for various reasons, including (1) the sample is too dilute, or (2) the sample was too small to start with.

The devices according to the present invention may be sterile, for example they may be sterilized by gamma irradiation. The devices are suitably packaged in a microorganismimpermeable container. It is envisaged that the devices according to the present invention will normally be disposable, single-use devices. For example, the housing, pivot arm and retaining clip (where present) may all be formed from injection molded thermoplastics.

These and other aspects of the present invention will now be described, by way of example, with reference to the accompanying drawings, in which:
Figure 1 is a perspective, partial cut-away view of a sample collection and testing device in accordance with the present invention, with an analytical test strip shown separated from the housing for illustrative purposes;
Figure 2 is a cross-sectional side elevation of the device of Figure 1, with a pivot arm shown in a sample collection configuration and with a retaining clip shown in a free configuration;
Figure 3 corresponds to Figure 2 but shows the pivot arm and the retaining clip being moved from their respective sample collection and free configurations; and
Figure 4 corresponds to Figures 2 and 3, but shows the pivot arm in a sample analysis configuration and the retaining clip in a retaining configuration.

With reference to the accompanying figures, a sample collection and testing device 10 generally comprises a housing 12, a pivot arm 14, a sample collector 16, a retaining clip 18 and an analytical test strip 20. The housing 12 is formed from an upper part 22 and a lower part 24. Each part may be made by injection molding of thermoplastics. The parts are fitted together by means of snap fittings 26. It will be appreciated, however, that the parts could additionally or instead be fitted together by means of adhesive.

An internal chamber 28 is defined between an upper surface of the lower housing part 26 and a lower surface of the upper housing part 22. The analytical test strip 20 is located within the chamber 28. The upper and lower housing parts further define a sample receiving port 29 in communication with the internal chamber.

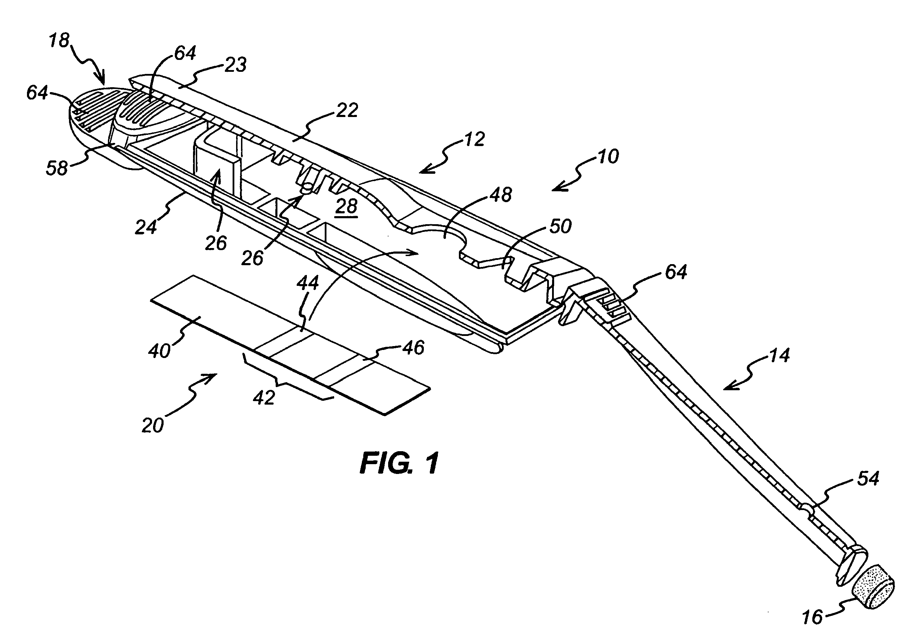

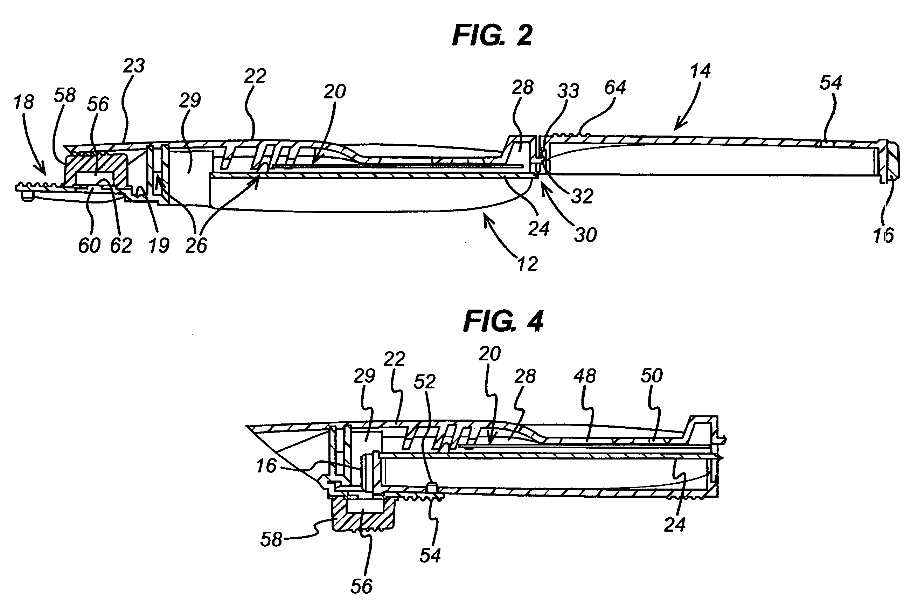

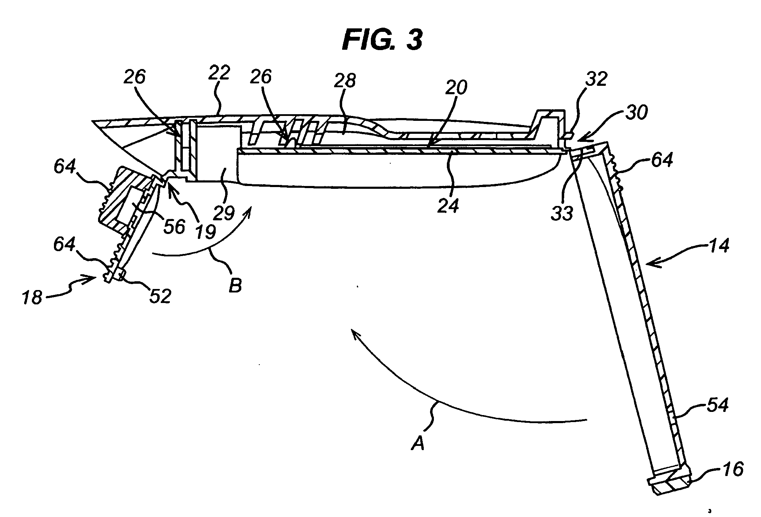

The pivot arm 14 has proximal and distal ends, the proximal end being pivotally attached to the housing 12 by hinge 30. The pivot arm is integral (molded in one piece) with the upper housing part 22. The hinge 30 is formed by a thin section at the junction between the upper housing part 22 and the proximal end of the pivot arm 14. It will be appreciated, however, that the pivot arm 14 may instead be formed integrally with the lower housing part 24 or be formed completely separately from the housing 12 and be attached thereto by any suitable pivotal hinge connection. A first detent 32 projects from a first end of the upper housing part 22. The first detent 32 engages second detent 33 at the proximal end of the pivot arm 14 to retain the pivot arm in a sample collection configuration shown in Figs 1 and 2, in which the pivot arm extends substantially coaxially with the principal axis of the housing. The engagement between the detents is in the nature of a snap-fitting, whereby application of a predetermined minimum pivoting torque is sufficient to overcome the engagement between the detents and allow pivoting of the pivot arm 14 as shown in Fig. 3.

The sample collector 16 comprises a swab formed from a hydrophilic sponge material, for example an open-celled hydrophilic polyurethane foam, which is mounted to the distal end of the pivot arm 14 by adhesive.

The analytical test strip 20 may comprise any reagents capable of indicating a positive test result when testing for a particular analyte in a test sample. In this embodiment, the test strip 20 is formed from a microporous cellulose acetate sheet and includes a detection zone 42 and a control zone 46. The detection zone 44 changes colour to indicate a positive test result. The control zone 46 changes colour to indicate that a test has been successfully completed thus to reduce the incidence of false positive results, in a manner well known in the art.

The housing 12 includes a window 48 in registration with the detection zone 44 to enable a user to view test results through the window. A second window 50 is also provided in the housing in registration with the control zone 46 to enable a user to check that the test has been completed successfully. It will be appreciated that there could instead be a single window enabling a user to view both the detection zone 44 and the control zone 46. The windows 48, 50 are suitably holes in the exterior of the housing 12. However, they could also be transparent portions of the housing.

The device according to this embodiment further comprises a retaining clip 18 having first and second ends, the first end being pivotally attached to the housing 12. The retaining clip 18 is integral with the lower housing part 24. A hinge 19 is formed at the junction between the first end of the clip and the lower housing part. It will be appreciated, however, that the retaining clip 18 may instead be formed integrally with the upper housing part 22 or be formed completely separately from the housing 12 and be attached thereto by any suitable connection, pivotal or otherwise. The retaining clip 18 has a free configuration, in which it lies in a plane substantially parallel to a plane defined by the interface between the upper and lower housing parts. See Figure 2.

The retaining clip includes a stud 52 at the second end, for receipt in a mating hole 54 near the distal end of the pivot arm 14. The retaining clip 18 is pivotable from the free configuration to a retained configuration in which the stud 52 is received in the pivot arm hole 54 in a friction fit, thus resisting movement of the pivot art 14 away from the sample analysis configuration.

A liquid reservoir 56 is defined by an internal chamber within a blister 58. The blister 58 is mounted on the retaining clip 18 above an aperture 60 in the retaining clip. The liquid reservoir 56 contains a predetermined volume of sterile saline solution. The solution may alternatively or additionally contain reagents for treating the sample collected by the device, for example immunological binding partners for selected analytes, buffers, or substrates for enzymes present in the sample. In certain embodiments the solution contains a binding partner or other inactivating agent for one or more interfering enzymes or other factors that may be present in the sample. The solution is held in the reservoir 56 by a rupturable membrane 62 that is placed across the aperture 60.

A portion 23 of the upper housing part 22 projects from the second end of the upper housing part. The projecting portion 23 protects the blister 58 when the retaining clip 18 is in the free configuration, to reduce the likelihood of premature release of liquid from the reservoir.

Operation of the device 10 is as follows. A user grasps the housing 12 in a hand with the pivot arm 14 in an initial, sample collection configuration. The sample collector 16 is used to obtain a sample. One example of a sample that would be suitable for analysis in the device 10 is wound fluid (exudate). It will be appreciated, however, that almost limitless varieties of samples could be collected and tested with the device. The samples may be biological or non-biological.

Once the sample has been collected, the user pivots the pivot arm 14 about the hinge 30 in the direction indicated by arrow A of Figure 3 until the sample collector 16 is received in the sample receiving port 29 as shown in Figure 4. This is defined as the sample analysis configuration. Since the sample collector 16 in this configuration is at least partially surrounded by portions of the housing defining the sample receiving port 29, the sample-containing sample collector is at least partially protected from contamination.

Next, the retaining clip 18 is pivoted in the direction indicated by arrow B of Figure 3 to the retaining configuration wherein the retaining clip stud 52 is frictionally received in the hole 54 in the pivot arm 14.

Once the pivot arm 14 has been moved to the sample analysis configuration and the retaining clip 18 has been moved into the retaining position, the blister 58 and hence the fluid reservoir 56 overlie the sample receiving port 29 and the sample-containing sample collector. The user presses the blister 58 to expel the solution from the reservoir 56 into the sample receiving port 29 along a flow path to the analytical device 20. The solution thus carries the sample from the sample-containing sample collector 16 to the analytical device 20, whereupon the sample is tested.

The device may be provided with raised ridges 64 or other indicia to indicate to a user where on the pivot arm 14 to push to move it from the sample collection configuration to the sample analysis configuration, where on the retaining clip 18 to push to move it from the free configuration to the retaining configuration and where to push to deform the blister 58. Suitably, the indicia includes symbols, such as numbers, to indicate the proper order in which to move or deform the pivot arm 14, retaining clip 18 and blister 58. For example, the ridges 10 on the pivot arm 14 may include the number 1, the ridges on the retaining clip 18 may include the number 2, and the ridges on the blister may include the number 3.

The present invention has been described above purely by way of example. It should be noted that modifications in detail may be made within the scope of the invention. For example, whereas the liquid reservoir 56 has been described as being located on a retaining clip 18, the reservoir could instead be located elsewhere on the device provided that a flow path existed between the reservoir 56, the sample receiving port 29 and the analytical device 20..

It is not essential for the device to include a retaining clip 18. However, its provision provides a suitable location for mounting the liquid reservoir 56 (where provided). A further advantage is that the retaining clip, when in the retaining configuration, further protects the sample-containing sample collector 16 from contamination.

Alternatively, in an embodiment where, in the sample analysis configuration, the sample collector 16 is brought directly into contact with the analytical device 20, it is not essential for the device to include a fluid reservoir.

Also, whereas the housing 12 has been described as including windows 48, 50 to view the detection strip 44 and the control line 46 of the analytical device 20, these could be omitted and a user instead remove the analytical device 20 from the housing 12 in order to view the test results.

Furthermore, whereas the housing 12 has been described as comprising upper and lower housing parts 22, 24, it will be understood that the housing could instead comprise a single part, or more than two parts. Whereas the parts have been described as being formed by injection moulding of thermoplastics, it will be understood that they could instead be formed by other suitable materials and by other suitable methods.

## Claims

1. A sample collection and testing device (10) comprising:
a housing (12) having side walls and a sample receiving port (29);
an analytical device in fluid communication with the sample receiving port (29);
a pivot arm (14) having proximal and distal ends, the proximal end pivotally attached to the housing (12);
a sample collector (16) mounted on the distal end of the pivot arm (14); and
wherein the pivot arm (14) is configured to pivote from a sample collection configuration in which the pivot arm (14) projects away from the housing (12) and the sample collector (16) is remote from the housing (12) to a sample analysis configuration in which pivot arm (14) is folded back along an outer surface of the housing (12) and the sample collector is located proximate to the sample receiving port (29); **characterised in that**
the device further comprises complementary detent elements (32, 33) associated respectively with the pivot arm (14) and the housing (12), wherein said complementary detent elements (32,33) are configured to retain the pivot arm (14) in the sample collection configuration until a predetermined minimum pivoting force is applied to the pivot arm (14) so that an engagement of the detent elements in overcome thereby allowing pivoting of the pivot arm.

2. A device according to claim 1 wherein the analytical device comprises an analytical test strip (20).

3. A device according to any preceding claim, wherein the housing includes a viewing window (48) for viewing a portion of the analytical device.

4. A device according to any preceding claim, wherein the proximal end of the pivot arm (14) is attached to the housing (12) by a hinge (30).

5. A device according to any preceding claim, wherein the housing (12) is an elongate housing having a principal axis, and the pivot arm (14), when in the sample collection configuration, is substantially coaxial with the said principal axis.

6. The device of any preceding claim further comprising a retaining clip (18) to retain the pivot arm (14) in the sample analysis configuration, wherein the retaining clip (18) is pivotally attached to the housing (12) and is pivotable from a free configuration to a retaining configuration in which at least a portion of the clip (18) overlies the distal end of the pivot arm (14) and the sample receiving port (29).

7. The device of any preceding claim, further comprising a liquid reservoir (56) for releasing a liquid onto the sample collector (16) when the device is in the sample analysis configuration.

8. The device of claim 7, wherein the liquid reservoir (56) is mounted on a retaining clip (18) as defined in claim 6.

## Patentansprüche

1. Probensammel- und -testvorrichtung (10), die Folgendes umfasst:
ein Gehäuse (12) mit Seitenwänden und einem Probenaufnahmeanschluss (29);
eine Analysevorrichtung in Fluidkommunikation mit dem Probenaufnahmeanschluss (29);
einen Schwenkarm (14) mit einem proximalen und einem distalen Ende, wobei das proximale Ende an dem Gehäuse (12) angelenkt ist; und
einen Probensammler (16), der an dem distalen Ende des Schwenkarms (14) montiert ist;
wobei der Schwenkarm (14) konfiguriert ist, aus einer Probensammelkonfiguration, in der der Schwenkarm (14) von dem Gehäuse (12) absteht und der Probensammler (16) von dem Gehäuse (12) entfernt ist, in eine Probenanalysekonfiguration, in der der Schwenkarm (14) längs einer äußeren Oberfläche des Gehäuses (12) zurückgeklappt ist und der Probensammler sich in der Nähe des Probenaufnahmeanschlusses (29) befindet, zu schwenken; **dadurch gekennzeichnet, dass**
die Vorrichtung ferner komplementäre Arretierungselemente (32, 33) umfasst, die dem Schwenkarm (14) bzw. dem Gehäuse (12) zugeordnet sind, wobei die komplementären Arretierungselemente (32, 33) konfiguriert sind, den Schwenkarm (14) so lange in der Probensammelkonfiguration zu halten, bis eine vorgegebene minimale Schwenkkraft auf den Schwenkarm (14) ausgeübt wird, damit ein Eingriff der Arretierungselemente überwunden wird, um dadurch das Schwenken des Schwenkarms zuzulassen.

2. Vorrichtung nach Anspruch 1, wobei die analytische Analysevorrichtung einen Analyseteststreifen (20) umfasst.

3. Vorrichtung nach einem vorhergehenden Anspruch, wobei das Gehäuse ein Beobachtungsfenster (48) aufweist, um einen Abschnitt der Analysevorrichtung zu beobachten.

4. Vorrichtung nach einem vorhergehenden Anspruch, wobei das proximale Ende des Schwenkarms (14) an dem Gehäuse (12) durch ein Scharnier (30) befestigt ist.

5. Vorrichtung nach einem vorhergehenden Anspruch, wobei das Gehäuse (12) ein lang gestrecktes Gehäuse mit einer Hauptachse ist und der Schwenkarm (14) dann, wenn er in der Probensammelkonfiguration ist, zu der Hauptachse im Wesentlichen koaxial ist.

6. Vorrichtung nach einem vorhergehenden Anspruch, die ferner eine Halteraste (18) umfasst, um den Schwenkarm (14) in der Probenanalysekonfiguration zu halten, wobei die Halteraste (18) an dem Gehäuse (12) angelenkt ist und aus einer freien Konfiguration in eine Haltekonfiguration, in der wenigstens ein Abschnitt der Raste (18) über dem distalen Ende des Schwenkarms (14) und dem Probenaufnahmeanschluss (29) liegt, schwenkbar ist.

7. Vorrichtung nach einem vorhergehenden Anspruch, die ferner einen Flüssigkeitsbehälter (56) umfasst, um eine Flüssigkeit auf den Probensammler (16) freizugeben, wenn die Vorrichtung in der Probenanalysekonfiguration ist.

8. Vorrichtung nach Anspruch 7, wobei der Flüssigkeitsbehälter (56) an einer Halteraste (18) wie in Anspruch 6 definiert montiert ist.

## Revendications

1. Dispositif (10) de collecte et de test d'échantillons comportant :
un boîtier (12) doté de parois latérales et d'un orifice (29) de réception d'échantillons ;
un dispositif analytique en communication fluidique avec l'orifice (29) de réception d'échantillons ;
un bras pivotant (14) présentant des extrémités proximale et distale, l'extrémité proximale étant en liaison pivot avec le boîtier (12) ;
un collecteur (16) d'échantillons monté sur l'extrémité distale du bras pivotant (14) ; et
le bras pivotant (14) étant configurable pour pivoter d'une configuration de collecte d'échantillons dans laquelle le bras pivotant (14) dépasse du boîtier (12) et le collecteur (16) d'échantillons est à l'écart du boîtier (12) à une configuration d'analyse d'échantillons dans laquelle le bras pivotant (14) est replié le long d'une surface extérieure du boîtier (12) et le collecteur d'échantillons est situé à proximité de l'orifice (29) de réception d'échantillons ;
**caractérisé en ce que** le dispositif comporte en outre des éléments complémentaires (32, 33) d'encliquetage associés respectivement au bras pivotant (14) et au boîtier (12), lesdits éléments complémentaires (32, 33) d'encliquetage étant configurés pour retenir le bras pivotant (14) dans la configuration de collecte d'échantillons jusqu'à ce qu'une force minimale prédéterminée de pivotement soit appliquée au bras pivotant (14) de façon à vaincre l'enclenchement des éléments d'encliquetage, permettant ainsi le pivotement du bras pivotant.

2. Dispositif selon la revendication 1, le dispositif analytique comportant une bandelette réactive analytique (20).

3. Dispositif selon l'une quelconque des revendications précédentes, le boîtier comprenant une fenêtre (48) de visualisation destinée à visualiser une partie du dispositif analytique.

4. Dispositif selon l'une quelconque des revendications précédentes, l'extrémité proximale du bras pivotant (14) étant rattachée au boîtier (12) par une articulation (30).

5. Dispositif selon l'une quelconque des revendications précédentes, le boîtier (12) étant un boîtier allongé présentant un axe principal et le bras pivotant (14), lorsqu'il se trouve dans la configuration de collecte d'échantillons, étant sensiblement coaxial audit axe principal.

6. Dispositif selon l'une quelconque des revendications précédentes, comportant en outre une agrafe (18) de retenue pour retenir le bras pivotant (14) dans la configuration d'analyse d'échantillons, l'agrafe (18) de retenue étant en liaison pivot avec le boîtier (12) et pouvant pivoter d'une configuration libre à une configuration de retenue dans laquelle au moins une partie du clip (18) recouvre l'extrémité distale du bras pivotant (14) et l'orifice (29) de réception d'échantillons.

7. Dispositif selon l'une quelconque des revendications précédentes, comportant en outre un réservoir (56) de liquide destiné à libérer un liquide sur le collecteur (16) d'échantillons lorsque le dispositif se trouve dans la configuration d'analyse d'échantillons.

8. Dispositif selon la revendication 7, le réservoir (56) de liquide étant monté sur une agrafe (18) de retenue telle que définie dans la revendication 6.
